# EUROPEAN PATENT APPLICATION

(11) **EP 1 010 800 A1**
(43) Date of publication of application: **21.06.2000**
(21) Application number: 98900217.5
(22) Date of filing: 13.01.1998
(51) Int. Cl.: D06M 15/01, D06M 23/08, A61L 15/20, A61L 15/50

(54) **SKIN-CONTACTING ARTICLES**

(30) Priority: 17.01.1997 JP 639197; 12.12.1997 JP 34278097
(71) Applicant: IDEMITSU PETROCHEMICAL CO., LTD., Tokyo 108-0014 (JP)
(72) Inventor: MIKAMI, Satoshi, Himeji-shi, Hyogo-ken 672 (JP); SANO, Masahiro, Himeji-shi, Hyogo-ken 672 (JP); YASUE, Takaharu, Himeji-shi, Hyogo-ken 672 (JP)
(74) Representative: Hucker, Charlotte Jane
(86) International application number: JP9800080
(87) International publication number: WO9831868

(57) **Abstract**

Natural organic impalpable powder is adhered to at least on a side in direct contact with the skin of a skin contact goods applied to skin. When the skin contact goods is, for instance, a sanitary goods having a surface material directly applied to the skin and an absorbing body covered with the surface material, the natural organic impalpable powder having an average particle size of less than 30µm is adhered by processing base of the surface member with a treatment. The treatment includes the natural organic impalpable powder and at least one of dispersion medium selected from the group consisting of water, organic solvent, resin emulsion and resin aqueous solution. By the above arrangement, moisture inhalation and touch of the surface material in direct contact with the skin can be improved.

## Description

### Technical Field

The present invention relates to a skin contact goods in contact with skin.

### Background Art

A sanitary goods such as disposable diaper and sanitary napkin being applied to skin has, for instance, an absorbing body for retaining fluid, surface material laminated onto one side of the absorbing body to be in direct contact with the skin and an impervious sheet laminated onto the other side of the absorbing body for preventing the fluid from leaking out of the absorbing body.

A synthetic fiber, non-woven fabric and the like are widely used as a base of the surface member of the sanitary goods. In recent years, characteristic such as permeability for immediately conducting excretions to the absorbing body, cleanliness and toughness against human movement are desired for the surface material. Non-woven fabric composed of polypropylene, polyester, polyethylene and the like is used as a material satisfying the above demand.

Similarly to the sanitary goods, tissue paper, wet tissue as well as make-up tools for putting on make-ups, specifically, puffs such as disposable puff and a powder puff, a make-up brush, sponge etc. are used as the skin contact goods in contact with the skin.

Since the skin contact goods are used in direct contact with or for rubbing the skin, a product having superior touch (texture) has been conventionally desired.

Especially, material such as polypropylene, polyethylene and polyester has bad touch (texture) and, furthermore, causes stuffiness on account of lack of moisture inhalation, which results in unpleasant feeling when they are applied to human body for a long time.

The object of the present invention is to provide a skin contact goods having good touch and moisture inhalation.

### Disclosure of the Invention

The present invention is a skin contact goods being used in contact with skin. The skin contact goods is characterized in having natural organic impalpable powder adhered at least on a side in direct contact with the skin.

The skin contact goods includes tissue paper, wet tissue, a mask and gauze etc. in addition to below-described sanitary goods and make-up tools.

The natural organic is, for instance, silk, collagen, cellulose, chitin, chitosan, wool, hemp, cotton, sponge powder, whey and the like. Any one of the natural organic or the combination thereof can be selectably used.

The natural organic impalpable powder can be obtained by, for instance, mechanically pulverizing the natural organic material to make into impalpable powder.

The natural organic impalpable powder can be adhered by dispersing the natural organic impalpable powder into aqueous resin such as aqueous macromolecule and resin emulsion, organic solvent such as alcohol and solvent such as water and by coating them. Incidentally, the aqueous resin or the solvent may be used alone or in combination.

The base to which the natural organic impalpable powder is adhered, i.e. the base composing the side of the skin contact goods in direct contact with the skin, may be, for example, fiber such as synthetic fiber and natural fiber, fabric base such as non-woven fabric, textiles and knits using the fibers, and synthetic resin film etc.

The synthetic resin may be, for instance, polypropylene, polyester, polyethylene and the like. The natural fiber may be, for instance, silk, cotton and the like.

The synthetic resin film may be, for instance, polyethylene film, polypropylene film, polyester film and the like.

Hydrophilic treatment, water repellant treatment, softening treatment, embossing, perforating may be applied to the base.

When the skin contact goods is a sanitary goods applied to the skin and has a surface material in direct contact with the skin and, as necessary, an absorbing body covered with the surface material, the surface material preferably includes a base having the natural organic impalpable powder adhered thereto.

The sanitary goods refers to articles applied to the skin for a predetermined time, which includes, for instance, disposable diaper, diaper liner, sanitary napkin and the like. Many of them have the absorbing body covered with the surface material.

The skin contact goods may also be a make-up tool used for make-ups.

The make-up tool includes, for instance, puff, sponge, make-up brush, face-mask and the like. The puff includes disposable puffs such as silk puff, cotton puff and rayon puff in addition to various make-up puffs for putting on make-up.

The natural organic impalpable powder preferably has an average particle size of less than 30µm.

When the average particle size exceeds 30µm, the natural organic impalpable powder can be hard to be adhered to the base and is likely to be felt rough.

In order to enhance the adhesion to the base, the natural organic impalpable powder preferably has an average particle size of less than 10µm.

The lower limit of the natural organic impalpable powder is preferably more than 0.1µm, more preferably more than 1µm.

When the average particle size is less than 1µm, the powder can be difficult to be handled. Furthermore, lots of work is necessary for pulverizing the natural organic material, thereby increasing production cost.

The natural organic impalpable powder is preferably adhered by being processed by a treatment including the natural organic impalpable powder. The treatment is preferably consisted of the natural organic impalpable powder and at least one dispersion medium selected from the group consisting of water,. organic solvent, resin emulsion and resin aqueous solution.

Since the natural organic is dispersed in the treatment, the natural organic impalpable powder can be uniformly adhered by the processing using the treatment.

The resin emulsion may be synthetic resin emulsion such as silicone type, polyurethane type, polyacryl type, fluorine type, polyvinyl alcohol type and carboxymethyl cellulose type, or, alternatively, natural resin emulsion.

The resin aqueous solution may be, for instance, synthetic resin aqueous solution including synthetic water-soluble macromolecule such as polyvinyl alcohol type and carboxymethyl cellulose type. Alternatively, natural resin aqueous solution including natural resin type collagen, gelatin, potassium alginate, natural gums and the like may also be used.

Surface active agent may be added to the treatment as necessary to improve the dispersion of the natural organic impalpable powder. The surface active agent may be, for instance, ionic surface active agent of anion and cation, non-ionic surface active agent such as polyethylene oxide derivative and sucrose fatty ester.

Thickening agent may be added in the treatment to prevent sedimentation of the natural organic impalpable powder. The thickening agent may be, for instance, cellulose derivative such as methyl cellulose and hydroxyethyl cellulose, and natural macromolecule thickening agent such as various type of gum, pectin, soda alginate, dextrin, agar and gelatin.

The processing with the treatment may be conducted by coating the treatment by gravure coating method, spray method, padding method (impregnation, dipping) and the like and by drying thereafter.

Incidentally, additional processing such as embossing may be conducted after coating and drying the treatment.

Content ratio of the natural organic impalpable powder in the treatment is preferably 0.5 to 50wt%.

When the content ratio of the natural organic impalpable powder is less than 0.5wt%, good touch and sufficient moisture inhalation sometimes may not be obtained. When the content ratio exceeds 50wt%, the treatment is likely to get too thick to be uniform, in other words, cause mass of powder.

When the dispersion medium is either one of the resin emulsion or the resin aqueous solution, solid resin content in the treatment is preferably 0.5 to 20wt%.

Since the resin works as a binder for fixing the natural organic impalpable powder to the base, the natural organic impalpable powder can be firmly adhered to the base through the resin.

When the solid resin content is below 0.5wt%, sufficient adhesion of the natural organic impalpable powder to the base by the resin may not be obtained. When the solid resin content exceeds 20wt%, good touch and moisture inhalation by the natural organic impalpable powder may not be obtained.

According to another aspect of the present invention, the present invention is a skin contact goods used in contact with skin, including natural organic impalpable powder adhered at least on a side in direct contact with the skin by processing using a treatment. The skin contact goods is characterized in that the treatment includes the natural organic impalpable powder having an average particle size of less than 30µm and either one of resin emulsion or resin aqueous solution for dispersing the natural organic impalpable powder, content ratio of the natural organic impalpable powder in the treatment is 0.5 to 50wt% and solid resin content in the treatment is 0.5 to 20wt%.

The skin contact goods may preferably be a sanitary goods applied to the skin and the skin contact goods preferably has a surface material in direct contact with the skin, the surface material including a base having the natural organic impalpable powder adhered thereto.

The skin contact goods may also preferably be a make-up tool used for make-ups.

### Brief Description of Drawings

Fig. 1 is a cross section showing first embodiment of the present invention; and
Fig. 2 is a cross section showing second embodiment of the preset invention.

### Best mode for Carrying out the Invention

Preferred embodiments of the present invention will be described below with reference to attached drawings.

### [First Embodiment]

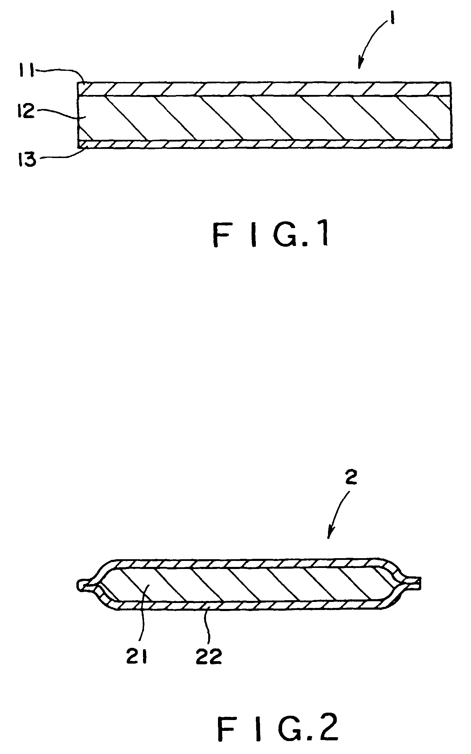

Fig. 1 shows sanitary goods 1 as a skin contact goods according to first embodiment.

The sanitary goods 1 is, for example, a disposable diaper which is used by applying to skin. The sanitary goods 1 has a surface material 11 in direct contact with the skin, an absorbing body 12 for absorbing and retaining fluid and a impervious sheet 13 for preventing the fluid from leaking.

The absorbing body 12 is covered with the surface member 11 on a side thereof facing the skin and absorbs the fluid through the surface member 11.

The surface member 11 has a base adhered with natural organic impalpable powders, which is obtained by processing the base with a treatment including the natural organic impalpable powders.

The treatment can be obtained by: adding the natural organic impalpable powders having average particle size of less than 30µm to an at least one dispersion medium selected from water, organic solvent, resin emulsion and resin aqueous solution so that the content of the natural organic impalpable powder is 0.5 to 50 wt%; stirring; and dispersing the natural organic impalpable powders. When the resin emulsion or the resin aqueous solution is used as the dispersion medium, the treatment is conditioned so that the solid resin content is 0.5 to 20wt%.

The treatment is coated to and dried on the base of surface material 11 for sanitary goods according to the present embodiment so that the natural organic impalpable powder is adhered.

### [Second Embodiment]

Fig. 2 shows a disposable cotton puff 2 as a skin contact goods according to second embodiment.

The cotton puff 2 is a make-up tool used for make-up. The cotton puff 2 has an intermediate cotton 21 and a surface member 22 covering the intermediate cotton 21. The surface member 22 is directly touched to the skin.

The surface member 22 is produced by adhering the natural organic impalpable powder to the base made of cotton in the same manner as in the first embodiment. In other words, the surface member 22 of the present embodiment can be obtained by processing the base using the same treatment as the first embodiment.

Incidentally, skin contact goods having the surface member, i.e. the sanitary goods 1 and the cotton puff 2, is described in the aforesaid embodiments. However, the arrangement of the skin contact goods to which the present invention is applied is not restricted to a surface member covering the area to which the skin touches.

In other words, the present invention may be applied to, for instance, a make-up brush as a make-up tool. In this case, the natural organic material may be adhered by conducting the processing by the aforesaid treatment to brush-hair as skin-contacting part.

When the present invention is applied to a face-mask as a make-up tool, a face-mask having superior touch can be obtained by adhering the natural organic material to at least one side of a base composed of non-woven fabric etc. touching the skin by conducting the processing of the aforesaid treatment.

The natural organic material may be adhered by conducting the aforesaid processing to the non-woven fabric, synthetic resin film structuring the surface member of the sanitary napkin as the sanitary goods.

Further, the tissue paper and the wet tissue as a skin contact goods can be made by adhering the natural organic material to the base of fiber sheet such as paper.

### [Experiment 1]

In the first embodiment, synthetic resin aqueous solution including polyvinyl alcohol [POVAL PVA-117 (trade name) manufactured by KURARAY Co. Ltd.] dissolved in water was used as the dispersion medium and silk power having average particle size of 5µm was used as the natural organic impalpable powder. The silk powder was mixed in the synthetic resin aqueous solution to be sufficiently dispersed, thereby obtaining the treatment of the present experiment.

The content ratio of respective component in the treatment is 10wt% of the silk powder, 85wt% of water and 5wt% of polyvinyl alcohol.

Polypropylene non-woven fabric was used as the base. The treatment was coated on the polypropylene non-woven fabric by a gravure processing machine so that treatment is coated by 5g/m² in Mezuke (Japanese weight unit showing weight per unit area). Subsequently, the polypropylene non-woven fabric was dried by a hot air drier at 80°C for one minute to obtain the surface material of the present experiment.

### [Experiment 2]

The natural organic impalpable powder of the experiment 1 was changed to collagen powder (average particle size of 4µm). The treatment was made in the same manner as the experiment 1 and the surface material for sanitary goods of the present experiment was obtained using the treatment.

### [Experiment 3]

The natural organic impalpable powder of the experiment 1 was changed to cellulose powder (average particle size of 6µm). The treatment was made in the same manner as the experiment 1 and the surface material for sanitary goods of the present experiment was obtained using the treatment.

### [Experiment 4]

The natural organic impalpable powder of the experiment 1 was changed to chitin powder (average particle size of 5µm). The treatment was made in the same manner as the experiment 1 and the surface material for sanitary goods of the present experiment was obtained using the treatment.

### [Experiment 5]

The natural organic impalpable powder of the experiment 1 was changed to chitosan powder (average particle size of 5µm). The treatment was made in the same manner as the experiment 1 and the surface material for sanitary goods of the present experiment was obtained using the treatment.

### [Experiment 6]

Synthetic resin emulsion including CL COAT 5400 [(trade name) manufactured by KYOEISHA CHEMICAL Co. Ltd.] dissolved in water was used as the dispersion medium of the experiment 1. The treatment was made in the same manner as in the experiment 1 to obtain the surface member for sanitary goods.

The content ratio of respective component in the treatment was 10wt% of silk powder, 60wt% of water and 30wt% of CL COAT 5400 (trade name). Since solid resin content of the CL COAT 5400 was approximately 20wt%, the solid resin content in the treatment was approximately 6%.

### [Experiment 7]

The natural organic impalpable powder of the experiment 6 was changed to wool powder (average particle size of 5µm). The treatment was made in the same manner as the experiment 6 and the surface material for the sanitary goods of the present experiment was obtained using the treatment.

### [Experiment 8]

The natural organic impalpable powder of the experiment 6 was changed to hemp powder (average particle size of 5µm). The treatment was made in the same manner as the experiment 6 and the surface material for the skin sanitary goods of the present experiment was obtained using the treatment.

### [Experiment 9]

The natural organic impalpable powder of the experiment 6 was changed to cotton powder (average particle size of 4µm). The treatment was made in the same manner as the experiment 6 and the surface material for the sanitary goods of the present experiment was obtained using the treatment.

### [Experiment 10]

The dispersion medium of the experiment 1 was changed to water. Polyethylene glycol ether as a surface active agent was added to the water to produce the treatment in the same manner as in the experiment 1. The surface material for the sanitary goods of the present experiment was obtained using the treatment.

The content ratio of respective components in the treatment was 10wt% of silk powder, 89.5wt% of water and 0.5wt% of polyethylene glycol ether.

### [Comparison 1]

The natural organic impalpable powder of the experiment 1 was changed to collagen powder (average particle size 35µm). The treatment was made in the same manner as in the experiment 1. The surface material for the sanitary goods of the present comparison was obtained using the treatment.

### [Comparison 2]

The content ratio of respective components of the treatment of the experiment 11 was set as 0.1wt% of silk powder, 99.4wt% of water and 0.5wt% of polyvinyl alcohol. The treatment was made in the same manner as in the experiment 1. The surface material for the sanitary goods of the present comparison was obtained using the treatment.

### [Comparison 3]

The content ratio of respective components of the treatment of the experiment 1 was set as 55wt% of silk powder, 44.5 wt% of water and 0.5wt% of polyvinyl alcohol. The treatment was made in the same manner as in the experiment 1. The surface material for the sanitary goods of the present comparison was obtained using the treatment.

### [Comparison 4]

The polyethylene non-woven fabric of the experiment 1 was used unprocessed without conducting processing by the treatment according to experiment 1.

The moisture inhalation and touch were evaluated for respective surface material for sanitary goods obtained in the experiments 1 to 10 and comparisons 1 to 4. The results are shown in Table 1 and 2.

The moisture inhalation was evaluated by measuring weight change of a sample which had reached to equilibrium at 23°C, 30%RH before being exposed in an atmosphere of 30°C, 80%RH for four hours with one side (powder-processed surface) upward.

The touch was evaluated by sensory test by ten persons. The touch of the surface material of the comparison 4 was set as standard (3 points), and the points were marked as: 5; superior touch, 4; good touch, 3; ordinary touch, 2; inferior touch, and 1; bad touch. Incidentally, the figures of Table 1 and 2 show average of the ten persons.

**Table 1**

| | Moisture Inhalation (g/m²) | Touch |
|---|---|---|
| Experiment 1 | 1.5 | 4.8 |
| Experiment 2 | 1.3 | 4.6 |
| Experiment 3 | 1.4 | 4.8 |
| Experiment 4 | 1.4 | 4.5 |
| Experiment 5 | 1.3 | 4.5 |
| Experiment 6 | 1.1 | 4.7 |
| Experiment 7 | 1.3 | 4.6 |
| Experiment 8 | 1.2 | 4.6 |
| Experiment 9 | 1.1 | 4.8 |
| Experiment 10 | 0.9 | 4.9 |

**Table 2**

| | Moisture Inhalation (g/m²) | Touch |
|---|---|---|
| Comparison 1 | 1.2 | 2.8 |
| Comparison 2 | 0.2 | 3.0 |
| Comparison 4 | 0.1 | 3.0 |

From the Table 1 and 2, it can be observed that the surface materials for sanitary goods of embodiments 1 to 10 were superior to the surface material of the comparison 4 and good and dry touch characteristic of natural material could be obtained, since the natural organic impalpable powder was adhered thereon.

On the other hand, since the average particle size of the natural organic impalpable powder of the surface material for sanitary goods according to comparison 1 was 35µm, which is more than 30µm, the touch was inferior on account of rough feeling.

Since the content ratio of the natural organic impalpable powder in the treatment was less than 0.5wt% and less natural organic impalpable powder was adhered to the base, moisture inhalation and touch of the surface material for sanitary goods according to comparison 2 could not be sufficiently improved.

Since the content ratio of the natural organic impalpable powder in the treatment for the surface material for sanitary goods of the comparison 3 was more than 50wt%, viscosity of the treatment was increased in manufacturing, so that the treatment gets non-uniform, i.e. generates powder mass and the treatment usable for the processing of the base material could not be obtained.

Since the surface material for the sanitary goods according to comparison 4 was unprocessed, it can be observed that the moisture inhalation and the touch were deteriorated.

### [Experiment 11]

In the second embodiment, synthetic resin aqueous solution including polyvinyl alcohol [POVAL PVA-117 (trade name) manufactured by KURARAY Co. Ltd.] dissolved in water was used as the dispersion medium and sponge power having average particle size of 5µm was used as the natural organic impalpable powder. The sponge powder was mixed in the synthetic resin aqueous solution to be sufficiently dispersed, thereby obtaining the treatment of the present experiment.

The content ratio of respective component in the treatment was 10wt% of the sponge powder, 85wt% of water and 5wt% of polyvinyl alcohol.

Cotton non-woven fabric was used as the base of the surface material. The treatment was coated on the cotton non-woven fabric by a spray processing machine so that treatment is coated by 30g/m² in Mezuke (Japanese weight unit showing weight per unit area). Subsequently, the cotton non-woven fabric was dried by a hot air drier at 150°C for one minute to obtain the surface material for cotton puff of the present experiment.

### [Comparison 5]

The cotton non-woven fabric of the experiment 11 was used unprocessed without conducting processing by the treatment of the experiment 11.

Moisture inhalation, touch and water absorption were evaluated for the respective surface material for the cotton puff obtained in the aforesaid experiment 11 and the comparison 5. The results are shown in Table 3.

The moisture inhalation and the touch were evaluated in the same manner as the aforesaid experiments 1 to 10 and the comparisons 1 to 4.

The water absorption was evaluated by dropping distilled water onto the surface of the surface material for cotton using a micropipet and measuring the amount of the dropped distilled water before the water leaked out of the back side of the surface material to measure the amount of absorbed water.

**Table 3**

| | Moisture Inhalation (g/m²) | Touch | Absorbed water (µl) |
|---|---|---|---|
| Experiment 11 | 6.8 | 4.8 | 540 |
| Comparison 5 | 8.2 | 4.2 | 124 |

According to Table 3, it can be observed that good moisture inhalation and good and dry touch characteristic of natural material as well as superior water absorption can be obtained by the surface material for cotton puff of the experiment 11, since the sponge powder as natural organic impalpable powder was adhered thereon.

Since the cotton puff is often used with liquid cosmetics and great water absorption is required, surface material suitable for the cotton puff can be obtained by adhering the sponge powder.

On the other hand, though the surface material for cotton puff according to the comparison 5 was superior in moisture inhalation and touch on account of being made of natural material of cotton non-woven fabric, the water absorption required as the surface material of the cotton puff could not be satisfied since it was unprocessed.

### Industrial Availability

As described above, the skin contact goods according to the present invention is suited for sanitary goods such as disposable diaper and sanitary napkin, and make-up tool such as make-up puff and make-up brush.

## Claims

1. A skin contact goods used in contact with skin, comprising natural organic impalpable powder adhered at least on a side in direct contact with the skin.

2. The skin contact goods according to Claim 1, wherein the skin contact goods is a sanitary goods applied to the skin, the skin contact goods comprising a surface material in direct contact with the skin, the surface material including a base having the natural organic impalpable powder adhered thereto.

3. The skin contact goods according to Claim 1, wherein the skin contact goods is a make-up tool used for make-ups.

4. The skin contact goods according to Claim 1, wherein the natural organic impalpable powder has an average particle size of less than 30µm.

5. The skin contact goods according to Claim 1, wherein the natural organic impalpable powder has an average particle size of less than 10µm.

6. The skin contact goods according to Claim 1, wherein the natural organic impalpable powder is adhered by being processed by a treatment including the natural organic impalpable powder, the treatment is consisted of the natural organic impalpable powder and at least one dispersion medium selected from the group consisting of water, organic solvent, resin emulsion and resin aqueous solution.

7. The skin contact goods according to Claim 6, wherein content ratio of the natural organic impalpable powder in the treatment is 0.5 to 50wt%.

8. The skin contact goods according to Claim 6, wherein the dispersion medium is either one of the resin emulsion or the resin aqueous solution; and wherein solid resin content in the treatment is 0.5 to 20wt%.

9. A skin contact goods used in contact with skin, comprising natural organic impalpable powder adhered at least on a side in direct contact with the skin by processing using a treatment,
wherein the treatment includes the natural organic impalpable powder having an average particle size of less than 30µm and either one of resin emulsion or resin aqueous solution for dispersing the natural organic impalpable powder;
wherein content ratio of the natural organic impalpable powder in the treatment is 0.5 to 50wt%; and
wherein solid resin content in the treatment is 0.5 to 20wt%.

10. The skin contact goods according to Claim 9, wherein the skin contact goods is a sanitary goods applied to the skin; and wherein the skin contact goods has a surface material in direct contact with the skin, the surface material including a base having the natural organic impalpable powder adhered thereto.

11. The skin contact goods according to Claim 9, wherein the skin contact goods is a make-up tool used for make-ups.
